# FASCICULE DE BREVET EUROPEEN

(11) **EP 1 635 882 B1**
(45) Date de publication et mention de la délivrance du brevet: **06.12.2006**
(21) Numéro de dépôt: 04767832.1
(22) Date de dépôt: 10.06.2004
(51) Int. Cl.: A61L 9/22

(54) **DISPOSITIF ET PROCEDE DE TRAITEMENT D'ODEURS PAR PLASMA FROID D'UN FLUX GAZEUX VICIE**
VORRICHTUNG UND VERFAHREN ZUR BEHANDLUNG VON GERÜCHEN EINES GASSTROMS MIT KALTPLASMA
DEVICE AND METHOD FOR TREATING ODOURS OF A STUFFY GAS STREAM WITH A COLD PLASMA

(30) Priorité: 12.06.2003 FR 0350211
(43) Date de publication de la demande: 22.03.2006
(73) Titulaire: Electricité de France (EDF), 75008 Paris (FR); Paganetti SA, 76600 Le Havre (FR)
(72) Inventeur: PARISSI, Ludovic, 75015 Paris (FR); TUVACHE, Frédéric, 77250 Moret sur Loing (FR); ADAM, Alain, 76600 Le Havre (FR)
(74) Mandataire: Poulin, Gérard
(86) Numéro de dépôt international: PCT/FR2004/050270
(87) Numéro de publication internationale: WO 2004/110510

(56) Documents cités:
- EP-A- 1 050 312
- GB-A- 420 360

## Description

### DOMAINE TECHNIQUE

La présente invention se rapporte à un dispositif et à un procédé de traitement d'odeurs par plasma froid d'un flux gazeux vicié.

### ETAT DE LA TECHNIQUE ANTERIEURE

Les domaines techniques concernés par les pollutions olfactives sont relativement nombreux. A titre d'exemples, on peut citer les élevages intensifs, l'industrie agro-alimentaire, les stations d'épuration, le traitement des déchets, ou encore les papeteries et l'industrie chimique.

De façon générale, les odeurs générées par ces activités- sont liées à l'émission de composés que l'on peut classer, pour les principaux, dans quatre familles chimiques.

A ce titre, on note les composés soufrés, du type H₂S, CH₃SH, les composés azotés, du type NH₃, CH₃NH₂, les aldéhydes, du type HCHO, CH₃CHO, et les acides gras volatiles, du type CH₃COOH, C₂H₅COOH. Bien entendu, d'autres composés peuvent être à l'origine d'odeurs, mais l'on trouve dans ces familles précitées ceux dont les seuils olfactifs sont les plus bas (0,001 à 5 mg/Nm³).

Bien que la plupart des composés à l'origine d'odeurs soient des composés organiques volatils (COV), la réglementation en matière d'odeurs est bien spécifique, et son évolution récente semble avoir pour conséquence de concerner un nombre de sites de plus en plus important.

Ainsi, dans le but de permettre aux exploitants de limiter leurs émissions d'odeurs afin de ne pas nuire au voisinage et de satisfaire les exigences réglementaires, outre la possibilité dont disposent ces exploitants de réduire les émissions à la source, ceux-ci nécessitent l'acquisition de dispositifs et de procédés fiables de traitement d'odeurs.

Parmi les solutions déjà proposées dans l'art antérieur, on connaît des installations offrant la possibilité de traiter séparément l'ensemble des polluants soumis à la législation. Cependant, cette technique est très difficile à mettre en oeuvre dans la mesure où dans la plupart des cas, les composés chimiques concernés se trouvent sous la forme de très faibles concentrations de multiples molécules odorantes diluées dans de très grandes masses d'air.

D'autre part, des technologies classiques de traitement ont également été testées, telles que l'absorption, la condensation, l'adsorption, l'oxydation biologique, ou encore l'oxydation thermique. Ces technologies se sont toutes avérées peu performantes, tant en termes de coût que d'efficacité.

En outre, pour faire face aux lacunes observées lors de la mise en oeuvre des solutions précitées, il a été proposé d'utiliser des technologies nouvelles telles que la photocatalyse, ou celle du plasma froid.

En effet, particulièrement bien adaptée à ce type de traitement, la technologie de plasma froid permet de canaliser l'énergie injectée vers le traitement des polluants olfactifs, contrairement à la technique d'oxydation thermique nécessitant de chauffer la totalité de l'effluent à traiter. Il en résulte alors un gain d'énergie considérable, notamment lorsque le milieu à traiter est fortement dilué.

Ainsi, de manière concrète, il a été présenté des dispositifs de traitement d'odeurs impliquant l'association d'un plasma froid avec un catalyseur, ou encore avec un ozoneur en opérant un séchage préalable de l'air afin de produire de grandes concentrations d'ozone.

Cependant, outre le fait que ces solutions ne se soient pas révélées totalement efficaces, leurs coûts est également très élevés, et les rendent par conséquent difficilement accessibles.

Le document EP-A-1 050 312 décrit un dispositif de traitement d'odeurs par plasma froid d'un flux gazeux vicié. Le dernier est mélangé avec un flux gazeux non-vicié et ensuite soumise à une étape d'humidification. Avant que le mélange gazeux ne quitte le dispositif, il est soumis à un plasma froid. Le dispositif selon GB 420 360 A dispose d'une entrée pour un flux gazeux vicié et d'une entrée pour un flux gazeux non-vicié. Le mélange flux gazeux vicié/ non-vicié humidifié est ionisé avant d'être acheminé vers un endroit utilisateur.

### EXPOSÉ DE L'INVENTION

L'invention a donc pour but de proposer un dispositif et un procédé de traitement d'odeurs par plasma froid d'un flux gazeux vicié, remédiant au moins partiellement aux inconvénients mentionnés ci-dessus relatifs aux réalisations de l'art antérieur.

Pour ce faire, l'invention a pour objet un dispositif de traitement d'odeurs par plasma froid d'un flux gazeux vicié, comportant un premier conduit à l'intérieur duquel un flux gazeux non-vicié est apte à être introduit et comprenant un humidificateur, un second conduit à l'intérieur duquel le flux gazeux vicié est apte à être introduit et comportant une chambre de mélange disposée en série avec des moyens primaires de génération d'un plasma, ainsi qu'un module intermédiaire communiquant avec les premier et second conduits et disposant de moyens secondaires de génération d'un plasma aptes à être traversés par le flux gazeux non-vicié humidifié provenant du premier conduit et se dirigeant vers la chambre de mélange du second conduit, le second conduit comprenant en outre une sortie d'un flux gazeux traité agencée en aval par rapport à la chambre de mélange et aux moyens primaires de génération d'un plasma.

Avantageusement, le dispositif de traitement d'odeurs selon l'invention utilise la technologie du plasma froid extrêmement fiable, tout en étant de conception simple et peu coûteux.

De plus, le principe même de l'invention consiste à faire transiter le flux gazeux non-vicié, préalablement humidifié, par les moyens secondaires de génération d'un plasma, avant qu'il ne pénètre à l'intérieur de la chambre de mélange du premier conduit. Avec une telle configuration, il se forme un second flux gazeux plasmogène très réactif, pouvant comprendre une forte concentration d'espèces du type O°, H₂O₂ et OH°, notamment en raison de l'humidification du flux gazeux non-vicié préalablement réalisée à l'aide de l'humidificateur.

Ainsi, dans la chambre de mélange du dispositif, le flux gazeux vicié à traiter entre en contact et réagit avec le second flux gazeux plasmogène réactif, ce qui provoque la modification de la structure des composés de ce flux vicié, et en modifie par conséquent l'odeur.

Il est par ailleurs noté que la réaction se produisant dans la chambre de mélange entre les flux gazeux vicié et plasmogène s'effectue après que le flux gazeux vicié ait transité par les moyens primaires de génération d'un plasma, ou avant que le mélange obtenu ait traversé ces mêmes moyens.

Ensuite, dans les deux cas de figure indiqués ci-dessus, le flux gazeux unique résultant prend la forme d'un flux gazeux traité qui est évacué du dispositif par la sortie prévue à cet effet, sur le second conduit.

Préférentiellement, le premier conduit comporte, d'amont en aval, une entrée du flux gazeux non-vicié, une vanne de régulation du débit d'entrée du flux gazeux non-vicié, des moyens de filtration du flux gazeux non-vicié, un échangeur de chaleur, l'humidificateur, des moyens d'aspiration du flux gazeux non-vicié, et une chambre d'alimentation communiquant avec le module intermédiaire, dans laquelle peut être introduit le flux de gaz non-vicié avant de pénétrer dans ce module.

On peut par ailleurs prévoir que le premier conduit comporte de plus, d'amont en aval et en aval de la chambre d'alimentation, une vanne de régulation de débit de sortie d'un premier flux gazeux plasmogène, des moyens tertiaires de génération d'un plasma, et une sortie du premier flux gazeux plasmogène.

Dans un tel cas, le flux gazeux non-vicié présent dans la chambre d'alimentation peut être divisé de manière à ce qu'une partie soit dirigée vers le module intermédiaire afin de générer le second flux gazeux plasmogène, et de façon à ce qu'une autre partie soit dirigée vers les moyens tertiaires de génération d'un plasma, afin de générer le premier flux gazeux plasmogène.

Ainsi, la sortie du premier flux gazeux plasmogène du premier conduit peut de préférence communiquer avec un local dans lequel se situe la source odorante générant le flux gazeux vicié. En effet, ce local permet alors avantageusement de remplir la fonction de chambre de réaction dans laquelle le premier flux gazeux plasmogène réagit avec le flux gazeux vicié présent dans le local, afin d'effectuer un prétraitement permettant d'assainir ce flux, et d'en améliorer par conséquent la qualité. A ce titre, il est noté que les espèces réactives présentes dans le local ne sont pas nocives. De cette façon, leur introduction à l'intérieur du local ne pose aucun problème pour ses occupants.

De manière préférentielle, le second conduit comporte, d'amont en aval, une entrée du flux gazeux vicié, une vanne de régulation du débit d'entré du flux gazeux vicié, des moyens de filtration du flux gazeux vicié, des moyens d'aspiration du flux gazeux vicié, la chambre de mélange communiquant avec le module intermédiaire, les moyens primaires de génération d'un plasma, et la sortie du flux gazeux traité.

Notons que cette disposition spécifique est retenue lorsque l'on désire mélanger le second flux gazeux plasmogène avec le flux gazeux vicié avant de faire transiter le mélange obtenu par les moyens primaires de génération d'un plasma.

Comme indiqué ci-dessus, lorsque l'on désire procéder différemment en faisant transiter le flux gazeux vicié par les moyens primaires de génération d'un plasma avant de le faire pénétrer dans la chambre de mélange du second conduit, cette dernière est alors située en aval par rapport aux moyens primaires de génération d'un plasma.

De préférence, le module intermédiaire comporte une pluralité de tuyères d'injection, chaque tuyère d'injection comportant une extrémité communiquant avec la chambre d'alimentation du premier conduit, une partie des moyens secondaires de génération d'un plasma, ainsi qu'au moins un injecteur débouchant dans la chambre de mélange du second conduit.

Ainsi, on peut alors prévoir que les premier et second conduits se situent l'un au-dessus de l'autre, et que les tuyères d'injection du module intermédiaire disposent chacune d'une portion d'injection partiellement délimitée par les moyens secondaires de génération d'un plasma et munie d'une pluralité d'injecteurs, la portion d'injection étant agencée de manière à épouser extérieurement la chambre de mélange du second conduit.

Toujours de façon préférentielle, les moyens primaires, secondaires et tertiaires de génération d'un plasma sont composés d'une pluralité de cellules unitaires de génération d'un plasma, disposées en série et/ou en parallèle, et du type grille-cylindre, fils-cylindre, ou encore pointes-cylindre.

En outre, l'invention a pour objet un procédé de traitement d'odeurs par plasma froid d'un flux gazeux vicié, ce procédé étant apte à être mis en oeuvre à l'aide d'un dispositif de traitement tel que celui décrit ci-dessus.

D'autres avantages et caractéristiques de l'invention apparaîtront dans la description détaillée non limitative ci-dessous.

### BRÈVE DESCRIPTION DES DESSINS

Cette description sera faite au regard des dessins annexés parmi lesquels ;
- la figure 1 représente une vue schématique en coupe d'un dispositif de traitement d'odeurs par plasma froid d'un flux gazeux vicié, selon un premier mode de réalisation préféré de la présente invention ;
- la figure 2 représente une vue en coupe prise le long de la ligne II-II de la figure 1 ;
- la figure 3 représente une vue agrandie en perspective d'une cellule unitaire de génération d'un plasma appartenant au module intermédiaire du dispositif représenté sur la figure 2 ; et
- la figure 4 représente une vue schématique en coupe d'un dispositif de traitement d'odeurs par plasma froid d'un flux gazeux vicié, selon un second mode de réalisation préféré de la présente invention.

### EXPOSÉ DÉTAILLÉ DE MODES DE RÉALISATION PRÉFÉRÉS

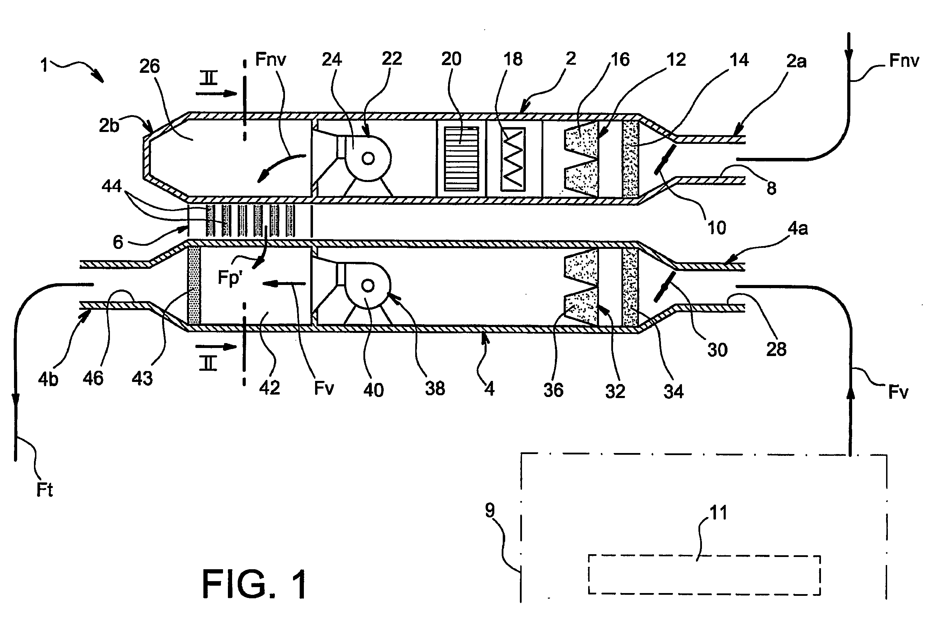

En référence à la figure 1, il est représenté un dispositif 1 de traitement d'odeurs par plasma froid d'un flux gazeux vicié Fv, selon un premier mode de réalisation préféré de la présente invention.

De manière générale, le dispositif de traitement 1 comporte un premier conduit 2, un second conduit 4, ainsi qu'un module intermédiaire 6 communiquant avec les deux conduits 2 et 4, ces trois éléments étant de préférence superposés de manière à former un bloc unique dans lequel le module intermédiaire 6 est au moins partiellement situé entre le premier conduit 2 et le second conduit 4. Notons que dans le premier mode de réalisation préféré représenté sur la figure 1, le premier conduit 2 se situe au-dessus du second conduit 4, parallèlement et à distance de ce dernier. Cependant, leur disposition pourrait bien entendu être tout autre, sans sortir du cadre de l'invention.

Le premier conduit 2 est préférentiellement de section carrée ou rectangulaire. Il dispose au niveau d'une extrémité amont 2a d'une entrée 8 d'un flux gazeux non-vicié Fnv, prenant par exemple la forme d'un flux d'air non-pollué prélevé en dehors du local 9 dans lequel se trouve la source odorante 11 générant le flux gazeux vicié Fv à traiter.

Plusieurs éléments sont ensuite disposés en série à l'intérieur de ce premier conduit 2. Parmi ces éléments, on trouve d'amont en aval dans une direction principale d'écoulement du flux gazeux non-vicié Fnv dans le premier conduit 2, une vanne de régulation 10 du débit d'entrée du flux gazeux non-vicié Fnv permettant de commander la quantité de gaz non-vicié introduite dans le premier conduit 2, puis des moyens de filtration 12 du flux gazeux non-vicié Fnv. Comme on peut le voir sur la figure 1, les moyens de filtration 12 destinés à filtrer les poussières comportent de préférence un pré-filtre 14, ainsi qu'un filtre à poches 16.

Ensuite, en aval du filtre à poches 16, le premier conduit 2 dispose d'un échangeur de chaleur 18, d'un humidificateur 20, ainsi que de moyens d'aspiration 22 du flux gazeux non-vicié Fnv, prenant préférentiellement la forme d'un ventilateur 24.

Par ailleurs, en aval du ventilateur 24, se trouve une chambre d'alimentation 26 dans laquelle le flux gazeux non-vicié Fnv peut être introduit par l'intermédiaire du ventilateur 24, cette chambre d'alimentation 26 s'étendant de préférence jusqu'à une extrémité aval 2b fermée du premier conduit 2, et communiquant avec le module intermédiaire.6.

Le second conduit 4 est également préférentiellement de section carrée ou rectangulaire, et sensiblement similaire au premier conduit 2. Il dispose au niveau d'une extrémité amont 4a, d'une entrée 28 du flux gazeux vicié Fv, prenant la forme d'un flux d'air pollué prélevé à l'intérieur du local 9 dans lequel se trouve la source odorante 11 générant le flux gazeux vicié Fv à traiter.

Plusieurs éléments sont ensuite disposés en série à l'intérieur de ce second conduit 4. Parmi ces éléments, on trouve d'amont en aval dans une direction principale d'écoulement du flux gazeux vicié Fv dans le second conduit 4, une vanne de régulation 30 du débit d'entré du flux gazeux vicié Fv permettant de commander la quantité de gaz vicié introduite dans le second conduit 4, puis des moyens de filtration 32 du flux gazeux vicié Fv. Comme on peut le voir sur la figure 1, les moyens de filtration 32 destinés à filtrer les poussières comportent de préférence un pré-filtre 34, ainsi qu'un filtre à poches 36.

En outre, en aval des moyens de filtration 32, se trouvent des moyens d'aspiration 38 du flux gazeux vicié Fv, prenant préférentiellement la forme d'un ventilateur 40.

Par ailleurs, en aval du ventilateur 40, se trouve une chambre de mélange 42 dans laquelle le flux gazeux vicié Fv peut être introduit par l'intermédiaire du ventilateur 40, cette chambre de mélange 42 communiquant avec le module intermédiaire 6.

Toujours en aval de cette chambre de mélange 42, le second conduit 4 est muni de moyens primaires 43 de génération d'un plasma, puis d'une sortie 46 d'un flux gazeux traité Ft.

Il est également noté qu'une solution alternative pourrait consister à inverser les positions de la chambre de mélange 42 et des moyens primaires 43 de génération d'un plasma, de façon à ce que ces derniers se situent en amont de la chambre de mélange 42.

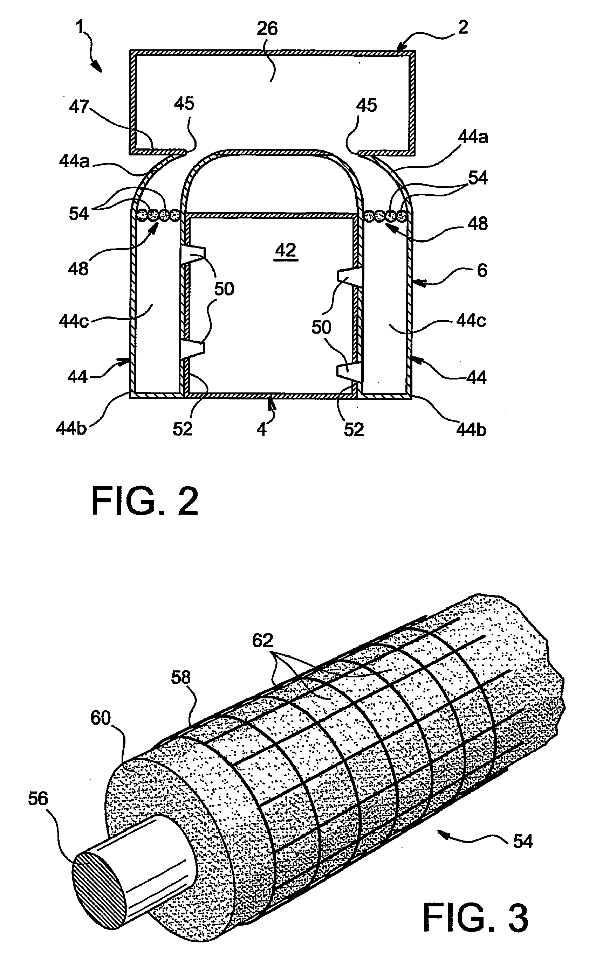

En référence à présent conjointement aux figures 1 et 2, on voit que le module intermédiaire 6 est réalisé de manière à communiquer simultanément avec le premier conduit 2 et le second conduit 4.

Pour ce faire, le module intermédiaire 6 comporte une pluralité de tuyères d'injection 44, dont chacune dispose d'une extrémité supérieure 44a communiquant avec la chambre d'alimentation 26 du premier conduit 2, par l'intermédiaire d'orifices 45 prévus dans une paroi inférieure 47 de ce même conduit 2. Notons à titre indicatif que la module intermédiaire 6 ou le premier conduit 2 peut être muni de vannes de régulation de débit (non représentées) afin de commander la quantité de gaz non-vicié introduite dans chaque tuyère d'injection 44. En outre, le module intermédiaire 6 comporte de préférence un système (non représenté) de répartition du flux gazeux non-vicié Fv provenant de la chambre d'alimentation 26.

D'autre part, le module intermédiaire 6 comprend des moyens secondaires 48 de génération d'un plasma, une partie de ces moyens secondaires 48 étant présente dans chaque tuyère d'injection 44, qui comporte également un système (non représenté) d'homogénéisation du flux de gaz la traversant.

Comme on peut l'apercevoir sur la figure 2, chaque tuyère d'injection 44 comporte une extrémité inférieure 44b, qui notamment à l'aide de la partie des moyens secondaires 48 de génération d'un plasma se situant dans cette même tuyère 44, délimite une portion d'injection 44c.

Chaque portion d'injection 44c est située en regard de la chambre de mélange 42 du second conduit 4, et dispose d'une pluralité d'injecteurs orientables 50 agencés en série et pénétrant à l'intérieur de cette chambre de mélange 42, en traversant des parois verticales 52 de cette dernière. De plus, on peut voir que les portions d'injection 44c des tuyères 44 sont agencées de manière à épouser extérieurement les deux parois verticales 52 de la chambre de mélange 42.

Il est précisé que dans ce premier mode de réalisation préféré de la présente invention, chacune des parois latérales 52 de la chambre de mélange 42 est associée à une pluralité de tuyères d'injection 44 disposées parallèlement et espacées les unes des autres. Cependant, le nombre et la disposition de ces tuyères 44 ainsi que le nombre, la disposition et l'orientation des injecteurs 50 peuvent être adaptés en fonction des besoins rencontrés, toujours de manière à assurer un mélange rapide et homogène entre le gaz provenant du premier conduit 2, et le gaz provenant du second conduit 4 du dispositif de traitement 1.

En référence conjointement aux figures 2 et 3, on voit que les moyens secondaires 48 de génération d'un plasma sont composés, dans chaque tuyère d'injection 44, d'une pluralité de cellules unitaires 54 de génération d'un plasma, agencées en série et/ou en parallèle.

Comme on peut mieux le voir sur la figure 2, dans chacune des tuyères d'injection 44, les cellules unitaires 54 sont disposées parallèlement dans un plan parallèle à la paroi inférieure 47 du premier conduit 2, de façon espacés les unes par rapport aux autres. A titre d'exemple indicatif, la distance entre deux cellules unitaires 54 directement adjacentes peut varier entre une valeur comprise entre 1 mm et 100 mm.

A ce titre, il est également précisé que chaque cellule unitaire 54, de préférence de forme cylindrique et du type grille-cylindre, fils-cylindre ou encore pointes-cylindre, dispose de deux électrodes métalliques. Ainsi, une des électrodes est reliée à la masse, tandis que l'autre électrode est reliée à une haute-tension alternative comprise entre environ quelques Volts et environ 100 kV. Enfin, il est indiqué que la fréquence du signal haute-tension est comprise entre environ 50 Hz et 500 kHz, et que chaque cellule unitaire 54 possède de préférence sa propre alimentation haute tension.

En référence plus spécifiquement à la figure 3, il est représenté une cellule unitaire 54 des moyens secondaires 48 de génération d'un plasma du module intermédiaire 6.

La cellule unitaire 54 représentée est du type grille-cylindre, à savoir qu'elle dispose d'une première électrode 56 en forme de cylindre, ainsi que d'une seconde électrode 58 en forme de grille située autour de la première électrode 56. Les deux électrodes 56 et 58 sont coaxiales et séparées par une couche diélectrique 60 d'épaisseur comprise entre environ quelques dixièmes de millimètre et environ 1 cm. La couche diélectrique 60 peut être du type verre, Pyrex^{®}, céramique, ou encore un intervalle gazeux.

De cette façon, les espaces 62 définis par les mailles de la grille formant la seconde électrode 58 sont des zones dans lesquelles se développe un plasma, lorsqu'un flux gazeux transite à proximité de ces cellules unitaires 52. Par conséquent, un flux gazeux plasmogène peut rapidement et facilement être créé à l'aide du flux gazeux non-vicié Fnv.

Bien entendu, les moyens primaires 43 de génération d'un plasma peuvent être conçus selon le même principe que celui décrit ci-dessus pour les moyens secondaires 48.

Le dispositif de traitement 1 est apte à fonctionner de la manière suivante.

Le flux gazeux non-vicié Fnv est tout d'abord introduit dans le premier conduit 2 par l'intermédiaire de l'entrée 8 à un débit donné, commandé par la vanne de régulation 10. Le flux gazeux non-vicié Fnv traverse alors successivement les moyens de filtration 12, l'échangeur de chaleur 18, l'humidificateur 20, le ventilateur 24, avant de se retrouver dans la chambre d'alimentation 26.

Ensuite, les vannes de régulation prévues au niveau de la chambre d'alimentation 26 ou sur le module intermédiaire 6 permettent de contrôler la quantité de gaz non-vicié humidifié provenant du premier conduit 2 et destinée à transiter par les moyens secondaires 48 de génération d'un plasma du module intermédiaire 6. De cette façon, une fois les moyens secondaires 48 traversés, cette quantité de gaz non-vicié contrôlée se transforme en un second flux gazeux plasmogène Fp' très réactif, pénétrant à l'intérieur de la chambre de mélange 42 du second conduit 4 par l'intermédiaire des injecteurs 50. Il est précisé que l'humidification préalable du flux gazeux non-vicié Fnv permet d'obtenir un second flux gazeux plasmogène Fp' disposant de fortes concentrations en espèces du type H₂O₂ et OH°, ces espèces étant avantageusement très réactives.

Simultanément, le flux gazeux vicié Fv est introduit dans le second conduit 4 par l'intermédiaire de l'entrée 28 à un débit donné, commandé par la vanne de régulation 30. Le flux gazeux vicié Fv traverse alors successivement les moyens de filtration 32, le ventilateur 40, avant de se retrouver dans la chambre de mélange 42, afin de s'y mélanger et de réagir avec le second flux gazeux plasmogène Fp'.

Ensuite, le mélange obtenu dans la chambre de mélange 42 transite par les moyens primaires 43 de génération d'un plasma, dans le but de transformer de façon plus significative les composés présents dans ce mélange, et par conséquent d'en réduire encore davantage leurs odeurs.

Lorsque le mélange est éjecté des moyens primaires 43, il génère la formation du flux gazeux traité Ft, qui peut alors être évacué de la sortie 46 prévue sur le second conduit 4.

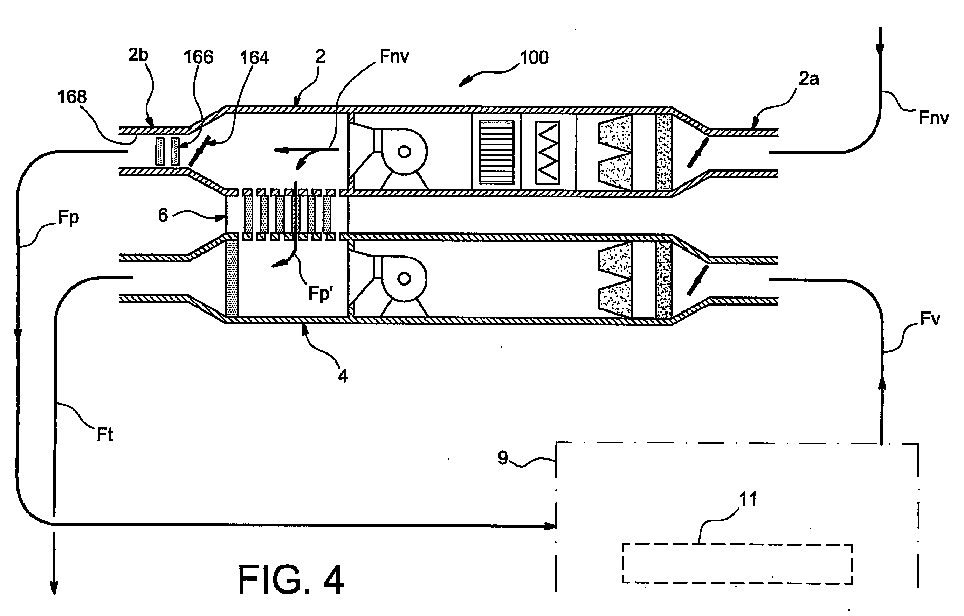

En référence à la figure 4, il est représenté un dispositif 100 de traitement d'odeurs par plasma froid d'un flux gazeux vicié Fv, selon un second mode de réalisation préféré de la présente invention.

Il est noté que sur la figure 4, les éléments portant les mêmes références numériques que celles attachées à des éléments représentés sur les figures 1 à 3, correspondent à des éléments identiques ou similaires.

Ainsi, on peut s'apercevoir que le dispositif de traitement 100 selon le second mode de réalisation préféré est relativement similaire au dispositif de traitement 1 selon le premier mode de réalisation préféré.

La différence principale structurelle réside dans la conception du premier conduit 2, et plus spécifiquement de celle de son extrémité aval 2b.

En effet, en aval de la chambre d'alimentation 26 du premier conduit 2, se trouve successivement une vanne de régulation 164 de débit d'un premier flux gazeux plasmogène Fp, des moyens tertiaires 166 de génération d'un plasma, puis enfin une sortie 168 du premier flux plasmogène Fp. Il est précisé que les moyens tertiaires 166 peuvent être de conception identique à celle des moyens secondaires 48, décrite précédemment.

Comme on peut le voir sur la figure 4, la sortie 168 communique avec le local 9 dans lequel se trouve la source odorante 11 générant le flux gazeux vicié Fv à traiter.

Avec un tel agencement, le dispositif de traitement 100 est apte à fonctionner de la manière suivante.

Tout d'abord, le flux gazeux non-vicié Fnv est introduit dans le premier conduit 2 jusqu'à la chambre d'alimentation 26, de manière similaire à celle rencontrée dans le dispositif de traitement 1.

Lorsque ce flux gazeux non-vicié Fnv arrive dans la chambre d'alimentation 26, une partie transite alors par la vanne de régulation 164 commandée afin de rejoindre les moyens tertiaires 166 de génération d'un plasma, tandis que l'autre partie du flux Fnv se dirige vers le module intermédiaire 6 de manière identique à celle décrite ci-dessus, dans le but de générer le second flux gazeux plasmogène Fp'.

Ainsi, la partie du flux gazeux non-vicié Fnv transitant par la vanne de régulation 164 et les moyens tertiaires 166 se transforme afin de générer la formation du premier flux gazeux plasmogène Fp également très réactif, ce dernier étant directement réinjecté dans le local 9, depuis la sortie 168 du premier conduit 2.

De cette façon, le local 9 dans lequel se trouve la source odorante 11 peut servir de chambre de réaction, ce qui permet d'assainir préalablement le flux gazeux vicié Fv présent dans le local 9, et d'en améliorer par conséquent la qualité.

Enfin, il est noté que le reste du fonctionnement de ce dispositif de traitement 100 est identique à celui du dispositif de traitement 1 décrit dans le premier mode de réalisation préféré de la présente invention, à la différence que le flux gazeux vicié Fv introduit dans la chambre de mélange 42 depuis l'entrée 28 du second conduit 4, correspond donc à un flux gazeux vicié prétraité à l'aide du premier flux gazeux plasmogène Fp.

Bien entendu, diverses modifications peuvent être apportées par l'homme du métier aux dispositifs 1 et 100 et procédés de traitement d'odeurs par plasma froid d'un flux gazeux vicié Fv qui viennent d'être décrits, uniquement à titre d'exemples non limitatifs.

## Revendications

1. Dispositif (1,100) de traitement d'odeurs par plasma froid d'un flux gazeux vicié (Fv), comportant un premier conduit (2) à l'intérieur duquel un flux gazeux non-vicié (Fnv) est apte à être introduit et comprenant un humidificateur (20), un second conduit (4) à l'intérieur duquel ledit flux gazeux vicié (Fv) est apte à être introduit et comportant une chambre de mélange (42) disposée en série avec des moyens primaires (43) de génération d'un plasma, ainsi qu'un module intermédiaire (6) communiquant avec les premier (2) et second conduits (4) et disposant de moyens secondaires (48) de génération d'un plasma aptes à être traversés par le flux gazeux non-vicié (Fnv) humidifié provenant du premier conduit (2) et se dirigeant vers la chambre de mélange (42) du second conduit (4), ledit second conduit (4) comprenant en outre une sortie d'un flux gazeux traité (Ft) agencée en aval par rapport à la chambre de mélange (42) et aux moyens primaires (43) de génération d'un plasma.

2. Dispositif (1,100) de traitement selon la revendication 1, **caractérisé en ce que** le premier conduit (2) comporte, d'amont en aval, une entrée (8) du flux gazeux non-vicié (Fnv), une vanne de régulation (10) du débit d'entrée du flux gazeux non-vicié (Fnv), des moyens de filtration (12) du flux gazeux non-vicié (Fnv), un échangeur de chaleur (18), l'humidificateur (20), des moyens d'aspiration (22) du flux gazeux non-vicié (Fnv), et une chambre d'alimentation (26) communiquant avec ledit module intermédiaire (6).

3. Dispositif (100) de traitement selon la revendication 2, **caractérisé en ce que** le premier conduit (2) comporte de plus, d'amont en aval et en aval de la chambre d'alimentation (26), une vanne de régulation (164) de débit de sortie d'un premier flux gazeux plasmogène (Fp), des moyens tertiaires (166) de génération d'un plasma, et une sortie (168) du premier flux gazeux plasmogène (Fp).

4. Dispositif (100) de traitement selon la revendication 3, **caractérisé en ce que** la sortie (168) du premier flux gazeux plasmogène (Fp) du premier conduit (2) communique avec un local (9) dans lequel se situe une source odorante (11) générant ledit flux gazeux vicié (Fv).

5. Dispositif (1,100) de traitement selon l'une quelconque des revendications précédentes, **caractérisé en ce que** le second conduit (4) comporte, d'amont en aval, une entrée (28) du flux gazeux vicié (Fv), une vanne de régulation (30) du débit d'entré du flux gazeux vicié (Fv), des moyens de filtration (32) du flux gazeux vicié (Fv), des moyens d'aspiration (38) du flux gazeux vicié (Fv), la chambre de mélange (42) communiquant avec ledit module intermédiaire (6), les moyens primaires (43) de génération d'un plasma, et la sortie (46) du flux gazeux traité (Ft).

6. Dispositif (1,100) de traitement selon l'une quelconque des revendications 1 à 4, **caractérisé en ce que** le second conduit (4) comporte, d'amont en aval, une entrée (28) du flux gazeux vicié (Fv), une vanne de régulation (30) du débit d'entré du flux gazeux vicié (Fv), des moyens de filtration (32) du flux gazeux vicié (Fv), des moyens d'aspiration (38) du flux gazeux vicié (Fv), les moyens primaires (43) de génération d'un plasma, la chambre de mélange (42) communiquant avec ledit module intermédiaire (6), et la sortie (46) du flux gazeux traité (Ft).

7. Dispositif (1,100) de traitement selon l'une quelconque des revendications 2 à 6, **caractérisé en ce que** le module intermédiaire (6) comporte une pluralité de tuyères d'injection (44), chaque tuyère d'injection (44) comportant une extrémité (44a) communiquant avec ladite chambre d'alimentation (26) du premier conduit (2), une partie des moyens secondaires (48) de génération d'un plasma, ainsi qu'au moins un injecteur (50) débouchant dans la chambre de mélange (42) du second conduit (4).

8. Dispositif (1,100) de traitement selon la revendication 7, **caractérisé en ce que** les premier et second conduits (2,4) se situent l'un au-dessus de l'autre, et **en ce que** les tuyères d'injection (44) du module intermédiaire (6) disposent chacune d'une portion d'injection (44c) partiellement délimitée par les moyens secondaires (48) de génération d'un plasma et munie d'une pluralité d'injecteurs (50), ladite portion d'injection (44c) étant agencée de manière à épouser extérieurement la chambre de mélange (42) du second conduit (4).

9. Dispositif (1,100) de traitement selon l'une quelconque des revendications 4 à 8 combinée à la revendication 3, **caractérisé en ce que** les moyens primaires (43), secondaires (48) et tertiaires (166) de génération d'un plasma sont composés d'une pluralité de cellules unitaires (54) de génération d'un plasma, disposées en série et/ou en parallèle.

10. Dispositif (1,100) de traitement selon la revendication 9, **caractérisé en ce que** chaque cellule unitaire (54) de génération d'un plasma est prise parmi le groupe constitué des cellules unitaires du type grille-cylindre, fils-cylindre et pointes-cylindre.

11. Procédé de traitement d'odeurs par plasma froid d'un flux gazeux vicié (Fv), **caractérisé en ce qu'**il est mis en oeuvre à l'aide d'un dispositif de traitement (1,100) selon l'une quelconque des revendications précédentes.

## Claims

1. Device (1, 100) for treatment of odours from a polluted flow of gas (Fv) by cold plasma, comprising a first conduit (2) inside which a non-polluted flow of gas (Fnv) is capable of being introduced and comprising a humidifier (20), a second conduit (4) inside which said polluted flow of gas (Fv) is capable of being introduced and comprising a mixing chamber (42) arranged in series with the primary plasma generation means (43), as well as an intermediary module 96) communicating with the first (2) and second conduits (4) and having secondary plasma generation means (48) capable of being crossed by the humidified non-polluted flow of gas (Fnv) coming from the first conduit (2) and moving toward the mixing chamber (42) of the second conduit (4), said second conduit (4) comprising additionally an outlet for a treated flow of gas (Ft) arranged downstream with respect to the mixing chamber (42) and the primary plasma generation means (43).

2. Treatment device (1, 100) according to claim 1, **characterized in that** the first conduit (2) comprises, from upstream to downstream, an inlet (8) for the non-polluted flow of gas (Fnv), a valve (10) for regulation of the inlet flow rate of the non-polluted flow of gas (Fnv), means for filtration (12) of the non-polluted flow of gas (Fnv), a heat exchanger (18), the humidifier (20), means for aspiration (22) of the non-polluted flow of gas (Fnv), and a feed chamber (26) communicating with said intermediary module (6).

3. Treatment device (100) as claimed in claim 2, **characterised in that** the first conduit (2) additionally comprises, from upstream to downstream and downstream of the feed chamber (26), a valve for regulation (164) of the outlet flow rate of a first plasmogenic flow of gas (Fp), tertiary plasma generation means (166), and an outlet (168) for the first plasmogenic flow of gas (Fp).

4. Treatment device (100) as claimed in claim 3, **characterised in that** the outlet (168) of the first plasmogenic flow of gas (Fp) of the first conduit (2) communicates with a premises (9) in which an odorous source (11) generating said polluted gas flow (Fv) is located.

5. Treatment device (1,100) as claimed in any of the preceding claims, **characterised in that** the second conduit (4) comprises, from upstream to downstream, an inlet (28) for the polluted flow of gas (Fv), a valve for regulation (30) of the inlet flow rate of the polluted flow of gas (Fv), means for filtration (32) of the polluted flow of gas (Fv), means for aspiration (38) of the polluted flow of gas (Fv), the mixing chamber (42) communicating with said intermediary module (6), the primary plasma generation means (43), and the outlet (46) for the treated flow of gas (Ft).

6. Treatment device (1,100) as claimed in any of claims 1 to 4, **characterised in that** the second conduit (4) comprises, from upstream to downstream, an inlet (28) for the polluted flow of gas (Fv), a valve for regulation (30) of the inlet flow rate of the polluted flow of gas (Fv), means for filtration (32) of the polluted flow of gas (Fv), means for aspiration (38) of the polluted flow of gas (Fv), the primary plasma generation means (43), the mixing chamber (42) communicating with said intermediary module (6), and the outlet (46) for the treated flow of gas (Ft).

7. Treatment device (1,100) as claimed in any of claims 2 to 6, **characterised in that** the intermediary module (6) comprises a plurality of injection nozzles (44), each injection nozzle (44) comprising an end (44a) communicating with said feed chamber (26) of the first conduit (2), a part of the secondary plasma generation means (48), as well as at least one injector (50) emerging into the mixing chamber (42) of the second conduit (4).

8. Treatment device (1,100) as claimed in claim 7, **characterised in that** the first and second conduits (2,4) are situated one above the other, and **in that** the injection nozzles (44) of the intermediary module (6) each possess an injection portion (44c) partially delimited by the secondary plasma generation means (48) and equipped with a plurality of injectors (50), said injection portion (44c) being arranged in such a way as to externally embrace the mixing chamber (420 of the second conduit (4).

9. Treatment device (1, 100) as claimed in any of claims 4 to 8 combined with claim 3, **characterized in that** the primary (43), secondary (48) and tertiary (166) plasma generation means are composed of a plurality of unit cells (54) for plasma generation, arranged in series and/or in parallel.

10. Treatment device (1, 100) as claimed in claim 9, **characterized in that** each unit cell (54) for plasma generation is taken from among the group made up of unit cells of the grid-cylinder, wire-cylinder and points-cylinder type.

11. Method for treatment of the odours from a polluted flow of gas (Fv) by cold plasma, **characterized in that** it is implemented by means of a treatment device (1, 100) as claimed in any of the preceding claims.

## Patentansprüche

1. Vorrichtung (1, 100) zur Behandlung von Gerüchen eines Abgasstroms (Fv) mittels Kaltplasma, umfassend eine erste Leitung (2), in deren Inneres ein Nichtabgasstrom (Fnv) eingebracht werden kann, und die einen Befeuchter (20) enthält, eine zweite Leitung (4), in deren Inneres der Abgasstrom (Fv) eingebracht werden kann, und die eine Mischkammer (42) enthält, welche in Reihe mit Primärmitteln (43) zur Erzeugung eines Plasmas angeordnet ist, sowie ein Zwischenmodul (6), welches mit der ersten (2) und der zweiten (4) Leitung in Verbindung steht und über Sekundärmittel (48) zur Erzeugung eines Plasmas verfügt, die dazu ausgelegt sind, von dem befeuchteten Nichtabgasstrom (Fnv) durchquert zu werden, der von der ersten Leitung (2) kommt und zur Mischkammer (42) der zweiten Leitung (4) gerichtet ist, wobei die zweite Leitung (4) ferner einen Ausgang für einen behandelten Gasstrom (Ft) enthält, der stromabwärts bezüglich der Mischkammer (42) und bezüglich der Primärmittel (43) zur Erzeugung eines Plasmas angeordnet ist.

2. Vorrichtung (1, 100) zur Behandlung gemäß Anspruch 1, **dadurch gekennzeichnet, dass** die erste Leitung (2) von stromaufwärts nach stromabwärts folgendes umfaßt: einen Eingang (8) für den Nichtabgasstrom (Fnv), ein Ventil (10) zur Regelung der Eintrittsrate des Nichtabgasstroms (Fnv), Mittel (12) zur Filterung des Nichtabgasstroms (Fnv), einen Wärmetauscher (18), den Befeuchter (20), Mittel (22) zum Ansaugen des Nichtabgasstroms (Fnv), sowie eine Versorgungskammer (26), die in Verbindung mit dem Zwischenmodul (6) ist.

3. Vorrichtung (100) zur Behandlung gemäß Anspruch 2, **dadurch gekennzeichnet, dass** die erste Leitung (2) ferner von stromaufwärts nach stromabwärts und stromabwärts der Versorgungskammer (26) folgendes umfaßt: ein Ventil (164) zur Regelung der Austrittsrate eines ersten plasmogenen Gasstroms (Fp), Tertiärmittel (166) zur Erzeugung eines Plasmas, und einen Ausgang (168) für den ersten plasmogenen Gasstrom (Fp).

4. Vorrichtung (100) zur Behandlung gemäß Anspruch 3, **dadurch gekennzeichnet, dass** der Ausgang (168) für den ersten plasmogenen Gasstrom (Fp) der ersten Leitung (2) in Verbindung mit einem Raum (9) ist, in dem sich eine Geruchsquelle (11) befindet, die den Abgasstrom (Fv) erzeugt.

5. Vorrichtung (1, 100) zur Behandlung gemäß einem der vorhergehenden Ansprüche, **dadurch gekennzeichnet, dass** die zweite Leitung (4) von stromaufwärts nach stromabwärts folgendes umfaßt: einen Eingang (28) für den Abgasstrom (Fv), ein Ventil (30) zur Regelung der Eintrittsrate des Abgasstroms (Fv), Mittel (32) zur Filterung des Abgasstroms (Fv), Mittel (38) zum Ansaugen des Abgasstroms (Fv), die Mischkammer (42), die in Verbindung mit dem Zwischenmodul (6) ist, die Primärmittel (43) zur Erzeugung eines Plasmas, sowie den Ausgang (46) für den behandelten Gasstrom (Ft).

6. Vorrichtung (1, 100) zur Behandlung gemäß einem der Ansprüche 1 bis 4, **dadurch gekennzeichnet, dass** die zweite Leitung (4) von stromaufwärts nach stromabwärts folgendes umfaßt: einen Eingang (28) für den Abgasstrom (Fv), ein Ventil (30) zur Regelung der Eintrittsrate des Abgasstroms (Fv), Mittel (32) zur Filterung des Abgasstroms (Fv), Mittel (38) zum Ansaugen des Abgasstroms (Fv), die Primärmittel (43) zur Erzeugung eines Plasmas, die Mischkammer (42), die in Verbindung mit dem Zwischenmodul (6) ist, sowie den Ausgang (46) für den behandelten Gasstrom (Ft).

7. Vorrichtung (1, 100) zur Behandlung gemäß einem der Ansprüche 2 bis 6, **dadurch gekennzeichnet, dass** das Zwischenmodul (6) eine Mehrzahl von Injektionsdüsen (44) umfaßt, wobei jede Injektionsdüse (44) ein Ende (44a) aufweist, das in Verbindung mit der Versorgungskammer (26) der ersten Leitung (2) ist, wobei ein Teil der Sekundärmittel (48) zur Erzeugung eines Plasmas sowie wenigstens ein Injektor (50) in die Mischkammer (42) der zweiten Leitung (4) münden.

8. Vorrichtung (1, 100) zur Behandlung gemäß Anspruch 7, **dadurch gekennzeichnet, dass** die erste und die zweite Leitung (2, 4) sich übereinander befinden, und dass jede der Injektionsdüsen (44) des Zwischenmoduls (6) über einen Injcktionsborosch (44c) verfügt, der teilweise von den Sekundärmi,tteln (48) zur Erzeugung eines Plasmas begrenzt und mit einer Mehrzahl von Injektoren (50) ausgestattet ist, wobei der Injektionsbereich (44c) derart angeordnet ist, dass er sich außen an die Mischkammer (42) der zweiten Leitung (4) anpaßt.

9. Vorrichtung (1, 100) zur Behandlung gemäß einem der Ansprüche 4 bis 8 in Kombination mit Anspruch 3, **dadurch gekennzeichnet, dass** die Primärmittel (43), die Sekundärmittel (48) und die Tertiärmittel (166) zur Erzeugung eines Plasmas aus einer Mehrzahl von einzelnen Zellen (54) zur Erzeugung eines Plasmas zusammengesetzt sind, die in Reihe und/oder parallel angeordnet sind.

10. Vorrichtung (1, 100) zur Behandlung gemäß Anspruch 9, **dadurch gekennzeichnet, dass** jede einzelne Zelle (54) zur Erzeugung eines Plasmas ausgewählt ist aus der Gruppe gebildet durch einzelne Zellen vom Typ Gitter-Zylinder, Drähte-Zylinder und Spitzen-Zylinder.

11. Verfahren zur Behandlung von Gerüchen eines Abgasstroms (Fv) mittels Kaltplasmas, **dadurch gekennzeichnet, dass** es mit Hilfe einer Vorrichtung zur Behandlung (1, 100) gemäß einem der vorhergehenden Ansprüche durchgeführt wird.
